(19) Europäisches Patentamt
European Patent Office
Office européen des brevets

(11) **EP 2 199 792 B1**

(12) **EUROPÄISCHE PATENTSCHRIFT**

(45) Veröffentlichungstag und Bekanntmachung des Hinweises auf die Patenterteilung:
**08.08.2012 Patentblatt 2012/32**

(51) Int Cl.:
***G01N 33/49*** *(2006.01)*

(21) Anmeldenummer: **08172121.9**

(22) Anmeldetag: **18.12.2008**

(54) **Verfahren zur Überprüfung der Qualität der thermischen Ankopplung einer Messzelle**

Method for testing the quality of the thermal coupling of a measuring cell

Procédé de surveillance de la qualité de l'accouplement thermique d'une cellule de mesure

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**23.06.2010 Patentblatt 2010/25**

(73) Patentinhaber:
• **F.Hoffmann-La Roche AG**
**4070 Basel (CH)**
Benannte Vertragsstaaten:
**AT BE BG CH CY CZ DK EE ES FI FR GB GR HR HU IE IS IT LI LT LU LV MC MT NL NO PL PT RO SE SI SK TR**
• **Roche Diagnostics GmbH**
**68305 Mannheim (DE)**
Benannte Vertragsstaaten:
**DE**

(72) Erfinder:
• **Steinböck, Wolf-Dietrich**
**8044 Graz (AT)**

• **Felsberger, Robert**
**8020 Graz (AT)**
• **Kraker, Michael**
**8044 Graz (AT)**
• **Schneider, Friedrich**
**8274 Buch/Hartberg (AT)**
• **Walla, Jürgen**
**8047 Graz (AT)**

(74) Vertreter: **Babeluk, Michael**
**Patentanwalt**
**Mariahilfer Gürtel 39/17**
**1150 Wien (AT)**

(56) Entgegenhaltungen:
**EP-A- 0 399 227       EP-A- 1 674 866**
**US-A- 5 232 667       US-A- 5 329 804**
**US-A- 5 832 921       US-A1- 2003 057 108**
**US-B2- 6 890 757**

**Beschreibung**

Die Erfindung betrifft ein Verfahren zur Überprüfung der Qualität der thermischen Ankopplung einer Messzelle an ein thermostatisierbares Element eines Analysators.

Die Messzelle oder auch Sensorkassette, die in einem Messkanal zumindest ein Sensorelement aufweist, ist in den Analysator, der eine thermostatisierbare Auflagefläche aufweist, austauschbar einsetzbar und zumindest in einem Kontaktbereich mit der thermostatisierbaren Auflagefläche in Kontakt bringbar. Für eine rasche, reproduzierbare Thermostatisierung der in der Messzelle vorliegenden Medien und Sensorelemente ist die Qualität der thermischen Kopplung zwischen dem thermostatisierbaren Element und der Messzellenwand von entscheidender Bedeutung. Ein zumeist unvermeidlicher Luftspalt zwischen thermostatisierbarem Element und Messzelle bzw. Sensorkassette muss innerhalb eines engen Toleranzfeldes bleiben.

Es ist bekannt, dass sensorische Elemente vieler Messeinrichtungen und Analysatoren temperaturabhängige Signaleigenschaften zeigen. Diese Temperaturabhängigkeit ist je nach Sensortyp durch die Beeinflussung chemischer Prozesse, deren Gleichgewichtslage und/oder deren Kinetik bedingt, oder wird insbesondere bei elektrochemischen Sensoren durch Änderung chemisch-physikalischer Eigenschaften hervorgerufen.

Derartige Sensoren werden häufig in medizinischen Analysensystemen zur Bestimmung von Gaspartialdrukken, des pH-Wertes oder der Ionen- und Metabolitkonzentrationen von Körperflüssigkeiten eingesetzt. Insbesondere werden solche Sensoren in Blutgasanalysatoren eingesetzt, welche in der medizinischen Diagnostik eine große Rolle spielen.

Während nun die Temperaturkoeffizienten der Sensoren relativ leicht durch entsprechende Kalibrationsmessungen ermittelt werden könnten, besteht ein Problem darin, dass beispielsweise bei der Bestimmung von Blutgasen und pH die Messgrößen ($pO_2$, $pCO_2$, pH) temperaturabhängig sind, und die für die Umrechnung erforderlichen Temperaturkoeffizienten der Probe nicht hinreichend genau bekannt sind. Eine Umrechnung der für eine Blutprobe, beispielsweise bei Raumtemperatur, gewonnenen Messwerte auf die bei Körpertemperatur (37°C) vorliegenden Werte ist daher ungenau.

Um die oben genannten Temperaturabhängigkeiten zu vermeiden, ist es bekannt, Messzellen mit Sensoren unter kontrollierten Temperaturverhältnissen - in Thermostaten - einzusetzen. Wenn Messzellen nach einer gewissen Einsatzzeit ausgetauscht werden sollen, ist für eine leichte Trennbarkeit zwischen der Messzelle einerseits und dem Thermostaten, der ein fester Bestandteil des Analysators ist, andererseits zu sorgen.

Im Allgemeinen werden die Messzellen in einer thermostatisierten Kammer des Analysators, welche auf konstanter Temperatur gehalten wird und zumeist aus synthetischen Werkstoffen gefertigt ist, betrieben.

Um die Situation im Körper des Patienten bestmöglich nachzubilden, wird hierbei bei einer Probentemperatur von 37°C gemessen. Auch wenn zwischen der Probennahme und dem Messzeitpunkt nur wenig Zeit vergeht, ist die Blutprobe deutlich abgekühlt und muss in der zumeist im Analysator vorliegenden Messzelle sehr rasch wieder auf Körpertemperatur gebracht werden.

Für eine rasche und reproduzierbare Thermostatisierung ist es wesentlich, neben den in die Messzelle eingebrachten Medien, wie Kalibriermedien, Kontrollmedien oder Probenflüssigkeiten, auch die in der Messzelle vorliegenden Sensoren schnellstmöglich und reproduzierbar auf die erforderliche Betriebstemperatur zu bringen.

In diesem Zusammenhang ist aus der US 5,046,496 A eine Sensoreinrichtung zur Messung der Blutgasparameter pH, $pCO_2$ und $pO_2$ bekannt geworden, bei welcher die einzelnen Elektroden mit Hilfe einer Dickfilmtechnik auf ein rechteckiges Trägerplättchen aus nicht leitender Keramik aufgetragen sind. Das Trägerplättchen mit den Messelektroden wird in das Gehäuse einer Durchflusszelle eingeklebt. Auf dem Trägerplättchen befinden sich weiters ein Temperatursensor und ein Heizelement, um die für die Messung notwendige Temperatur bereitzustellen und zu regeln.

Aus der US 6,890,757 B2 ist ein portables Diagnosesystem bekannt, bei welchem das Heizelement ebenfalls direkt in den Sensorchip mit den einzelnen Elektroden integriert ist, wobei der Sensorchip zur Temperaturmessung von einem IR-Sensor berührungslos abgetastet wird.

Schließlich ist aus der US 2003/0057108 A1 ein Verfahren zur schnellen Hydratisierung und Erwärmung von chemischen, elektrochemischen und biochemischen Sensoren bekannt. Die Sensorkassette besteht aus einem Unterteil aus Kunststoff, in welchem die Sensoren angeordnet sind und einer Abdeckplatte aus Metall, welche auch zur Übertragung von Wärme in die Sensorkassette verwendet werden kann. Zu diesem Zweck ist die Abdeckplatte mit entsprechenden Heiz- oder Kühlelementen, beispielsweise einem Peltierelement, kontaktiert.

Nachteilig bei den vorgenannten Anordnungen ist dabei der zusätzliche Aufwand, der mit der Integration eines Heizelementes sowie eines Temperaturmesselementes direkt in die Messzelle verbunden ist.

Aus der EP 1 367 392 B1 ist ein Analysengerät mit einer thermostatisierbaren Messzelle bekannt, die nicht näher beschriebene elektrochemische Elektroden aufweist. Die Messzelle wird mit Peltierelementen thermostatisiert, wobei zwischen den Peltierelementen und der Messzellenwand ein flaches, thermisch leitendes Verteilerelement angeordnet ist. Die so gewählte Thermostatisierung kommt daher aufgrund der unvermeidbaren Luftspalte einem Luftbad gleich, so dass der Wärmeübergang in der Hauptsache durch die Dicke des schlecht wärmeleitenden polymeren Werkstoffes um den elektrochemischen Sensor und der verbleibenden Luftspalte zur thermostatisierten Oberfläche begrenzt wird.

Zur Verbesserung des Wärmeüberganges auf die Messzelle ist aus der EP 1 674 866 eine wärmeleitfähige, elastische oder plastische Schicht bekannt, welche zumindest im Kontaktbereich an zumindest einer Messzellenwand oder der thermostatisierbaren Auflagefläche des Analysators haftet und beim Austausch der Messzelle von der gegenüberliegenden thermostatisierbaren Auflagefläche oder Messzellenwand weitgehend rückstandsfrei entfernbar ist. Weiters wird vorgeschlagen, dass die Messzellenwand, an deren dem Messkanal zugewandten Innenseite ein oder mehrere Sensorelemente angeordnet sind, zumindest im Kontaktbereich mit der thermostatisierbaren Auflagefläche des Analysators aus einem wärmeleitfähigen Metall oder einer Metalllegierung besteht.

Durch derartige Maßnahmen (wärmeleitfähige Schicht bzw. metallische Messzellenwand), welche auch kombiniert werden können, wird der Wärmedurchgangswiderstand von der Wärmequelle, der thermostatisierten Auflagefläche des Analysators, bis zur Sensor- bzw. Probenebene wesentlich minimiert.

Bei Vorliegen einer schadhaften Messzelle oder einer größeren Verunreinigung der Kontaktflächen ist die Berührung der thermostatisierten Oberfläche nur an wenigen Punkten und auf unreproduzierbare Art und Weise gegeben, so dass die Qualität der thermischen Ankopplung leidet, ohne dass diese Fehlfunktion rasch erkannt werden kann.

Die Folge einer unzureichenden Ankopplung ist eine verzögerte Temperaturanpassung des Sensors und der Probe. Damit ist die Messbereitschaft bei der jeweiligen Solltemperatur verlangsamt, bzw. eine Messung könnte zu früh erfolgen, noch bevor die Messtemperatur von z.B. 37°C erreicht ist.

Aufgabe der Erfindung ist es, ausgehend vom dargelegten Stand der Technik, ein Verfahren zur Kontrolle der thermischen Ankopplung einer in einen Analysator einsetzbaren Messzelle vorzuschlagen, welche möglichst unter Verzicht auf ein Heizelement und einen Temperatursensor in der Messzelle auskommt, wobei ein Fehler der thermischen Ankopplung beim Wechsel der Messzelle, bei einer Fehlfunktion oder während der Einsatzzeit der Messzelle rasch und eindeutig nachweisbar sein soll.

Die erfindungsgemäße Lösung weist folgende Schritte auf:

a) Einsetzen der Messzelle in den Analysator, wobei ein mechanischer Kontakt zum thermostatisierbaren Element hergestellt wird;

b) Befüllen des Messkanals mit einer vom Analysator bereitgestellten, internen Flüssigkeit, wie Kalibrierflüssigkeit, Waschlösung, etc. oder mit einer externen Flüssigkeit, wie Probenflüssigkeit, Qualitätskontrollflüssigkeit, etc.;

c) Abwarten eines Temperaturausgleichs zwischen interner oder externer Flüssigkeit und Messkanal;

d) Applizieren einer raschen Temperaturänderung mit Hilfe des thermostatisierbaren Elementes;

e) Messen eines zeitlichen Signalverlaufes an zumindest einem Sensorelement der Messzelle nach dem Applizieren der raschen Temperaturänderung; und

f) Bestimmung der Qualität der thermischen Ankopplung aus einer Analyse des zeitlichen Signalverlaufes der Messung gemäß Schritt e).

Erfindungsgemäß kann nun in Punkt e) der zeitliche Signalverlauf einer temperaturabhängigen Größe der internen oder der externen Flüssigkeit oder der zeitliche Signalverlauf eines temperaturabhängigen, elektrochemischen oder optischen Sensorelements gemessen werden.

Das Abwarten des Temperaturausgleichs zwischen interner oder externer Flüssigkeit und Messkanal in Verfahrensschritt c) kann beispielsweise dadurch erfolgen, dass eine vorbestimmte Zeit abgewartet wird, innerhalb welcher dieser Temperaturausgleich stattfinden kann. In einer alternativen Ausführungsform kann auch in Verfahrenschritt c) der zeitliche Signalverlauf einer temperaturabhängigen Größe der internen oder der externen Flüssigkeit oder der zeitliche Signalverlauf eines temperaturabhängigen, elektrochemischen oder optischen Sensorelements gemessen und analysiert werden, um aus dessen Verlauf das Erreichen des Temperaturausgleichs zwischen interner oder externer Flüssigkeit und Messkanal in Verfahrensschritt c) zu bestimmen. Dies kann beispielsweise dadurch erfolgen, indem die Zeit abgewartet wird, bis sich der zeitliche Verlauf dieses Signals nicht mehr wesentlich ändert.

Im Fall von Blutanalysatoren ist beispielsweise die elektrische Leitfähigkeit der Betriebsflüssigkeiten (Kalibrierflüssigkeiten, Waschlösungen, Qualitätskontrollflüssigkeiten) und der Probe (Blut) temperaturabhängig. Die Gaspartialdrücke ($PO_2$ und $PCO_2$) und der pH-Wert sind ebenfalls temperaturabhängig. Die zu messenden Konzentrationen, insbesondere die Ionen- und Metabolitkonzentration sind in Regel nicht temperaturabhängig. Zumindest ist in weiten Bereichen die Temperaturabhängigkeit so gering, dass sie nicht berücksichtigt werden muss. In diesen Fällen kann nun die Temperaturabhängigkeit des elektrochemischen oder optischen Sensorelements selbst herangezogen werden.

Von besonderem Vorteil ist es, wenn zur Messung des zeitlichen Signalverlaufes die für die Probenmessung bereits vorhandenen Sensorelemente der Messzelle eingesetzt werden. Es erfolgt somit eine völlig automatische und somit ökonomische Überprüfung der thermischen Ankopplung der Sensorkassette mittels vorhandener Sensorik.

In einer bevorzugten Ausführungsform erfolgt die Überprüfung der thermischen Ankopplung beispielsweise dadurch, dass der zeitliche Signalverlauf der Leitfähigkeit, des Widerstandes oder der Impedanz (oder eines davon abgeleiteten Parameters) der Betriebsflüssigkeit gemessen wird.

Eine bevorzugte Messmethode, nämlich die Leitfähigkeitsmessung, ist sehr einfach durchzuführen. Im Fall der Leitfähigkeitsmessung bestehen die "sensorischen Elemente" im Prinzip aus einfachen elektrischen Kontakten im Messkanal. Im Fall der Leitfähigkeitsmessung weisen die sensorischen Elemente selbst (in erster Näherung) keine nennenswerte Temperaturabhängigkeit auf. Die Messgröße selbst, nämlich der elektrische Leitwert der Flüssigkeit im Messkanal, ändert sich im Wesentlichen proportional mit deren Temperatur, so dass der zeitliche Signalverlauf direkt (d.h. ohne Verzögerung) mit dieser Temperatur korreliert.

Bei chemischen Sensoren kann der zeitliche Signalverlauf noch von der temperaturabhängigen Änderung von Konzentrationswerten der zu bestimmenden Substanz überlagert sein, beispielweise wenn der Sensor bei einer gegebenen Temperatur nicht im Äquilibrium mit der zu bestimmenden Substanz ist, muss eine gewisse Ansprechzeit abgewartet werden.

Gemäß einer Ausführungsvariante ist es auch möglich, dass der zeitliche Verlauf des Messsignals eines in der Messzelle befindlichen elektrochemischen oder optischen Sensorelementes zur Bestimmung einer in der Probeflüssigkeit vorliegenden Substanz, welches sich in Kontakt mit der internen oder externen Flüssigkeit befindet, nach Applikation eines Temperatursprungs gemessen wird.

Besonders bevorzugte Sensorelemente sind elektrochemische oder optische Sensorelemente zur Bestimmung von Ionen, beispielsweise Kationen wie $Li^+$, $Na^+$, $K^+$, $Mg^{++}$, $Ca^{++}$ oder Anionen wie $Cl^-$. In den verwendeten Flüssigkeiten, z.B. Kalibrier- oder Qualitätskontrollflüssigkeiten liegen diese Stoffe beispielsweise in Form gelöster Salze (LiCl, NaCl, KCl, $MgCl_2$, $CaCl_2$) vor.

Weitere bevorzugte Sensorelemente sind elektrochemische oder optische Sensorelemente zur Bestimmung von in einer Probe vorliegenden biochemischen Substanzen wie z.B. Glukose, Laktat, Harnstoff, Creatinin. Diese Sensorelemente könnte beispielsweise verwendet werden, wenn keine der oben genannten Sensorelemente in der Messzelle vorliegen.

Weiters können elektrochemische und optische Sensorelemente zur Bestimmung von in einer Probe gelösten Gasen wie z.B. Sauerstoff und Kohlendioxid oder elektrochemische und optische Sensorelemente zur Bestimmung des pH-Werts verwendet werden. Derartige Sensorelemente unterscheiden sich in der Regel von den eingangs genannten Sensorelementen dadurch, dass sowohl Sensorelement als auch der Werte der in der Flüssigkeit gelösten Substanzen selbst, z.B. deren Partialdruck oder die $H^+$-Ionenkonzentration, temperaturabhängig sind.

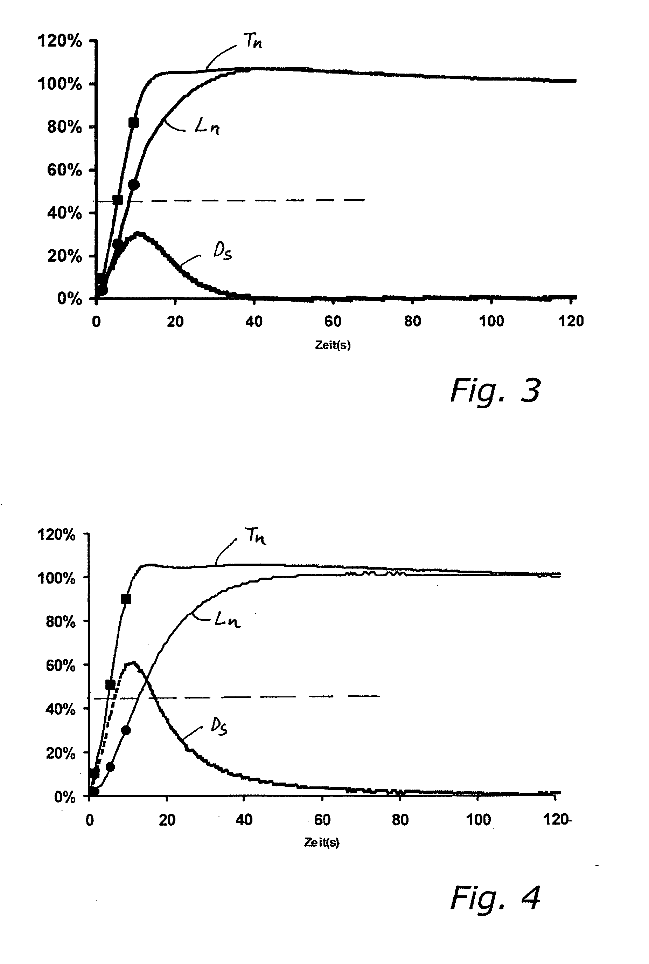

Von besonderem Vorteil ist es, wenn der zeitliche Verlauf der temperaturabhängigen Größe der internen oder externen Flüssigkeit mit dem zeitlichen Verlauf der Temperatur des thermostatisierbaren Elementes verglichen wird. Die entsprechenden Messkurven können beispielsweise normiert und der zeitliche Verlauf eines Differenzwertes oder deren Quotient ermittelt werden (siehe Fig. 3 bis Fig. 6). Es können aber auch andere Auswerteverfahren des zeitlichen Verlaufs, beispielsweise durch Bestimmen von Ableitungen, verwendet werden.

Bei der Durchführung des erfindungsgemäßen Verfahrens wird über ein geräteseitiges Heizelement nach dem Befüllen der Messzelle mit einem flüssigen Medium (Kalibriermedium, Qualitätskontrollmedium, Waschflüssigkeit oder Probenflüssigkeit) die Temperatur der geräteseitigen Auflagefläche der Messzelle möglichst sprunghaft verändert. Dabei spielt - in gewissen Bereichen - weder die Sprungamplitude noch die Richtung (Erwärmen oder Abkühlen) eine wesentliche Rolle. Parallel hierzu wird z.B. die Leitfähigkeit über die Zeit mit den in der Messzelle vorhandenen Leitfähigkeitselektroden gemessen.

Über die Kinetik der Leitfähigkeitsänderung in Antwort auf den geräteseitig applizierten Temperatursprung (z.B. durch Bestimmung der Steilheit der Messkurven oder Bewertung eines hystereseähnlichen Parameters zwischen Temperaturverlauf der Auflagefläche und Leitfähigkeitsänderung) können nun Aussagen über die Güte der thermischen Ankopplung gemacht werden.

Da in den meisten Fällen nicht die tatsächliche Temperatur, sondern nur ein Parameter, welcher mit dem Temperaturverlauf korreliert, erfasst werden muss, kann eine bereits vorhandene Sensorik (z.B. Elektroden zur Bestimmung des Hämatokritwertes mittels Messung der elektrischen Leitfähigkeit der Blutprobe) genutzt werden und Kosten für einen zusätzlichen in der Messzelle integrierten Temperatursensor eingespart werden.

Neben den Elektroden zur Bestimmung der Leitfähigkeit können erfindungsgemäß auch andere in der Messzelle vorhandene sensorische Elemente verwendet werden, beispielsweise elektrochemische oder optische Sensoren zur Bestimmung der Gaspartialdrucke (z.B. $PO_2$, $PCO_2$), des pH-Werts, der Elektrolyte ($Li^+$, $K^+$, $Na^+$, $Mg^{++}$, $Ca^{++}$, $Cl^-$) und der Metabolite (z.B., Glukose, Laktat, Harnstoff, Creatinin) in Körperflüssigkeiten, insbesondere Blutproben.

Besonders bevorzugte sensorische Elemente sind elektrochemische und optische Sensoren zur Bestimmung der Elektrolytwerte. Optische Sensoren arbeiten mit Farbstoffen, deren optische Eigenschaften (z.B., Absorption, Lumineszenz) temperaturabhängig sind.

Elektrochemische Sensoren zur Bestimmung der Elektrolytwerte sind zumeist potentiometrische Elektroden. Gemessen wird die Potentialdifferenz EMF (electromotive force) zwischen einer Referenzelektrode und einer ionenselektiven Messelektrode. Die EMF in Abhängigkeit der Konzentration [c] bzw. Aktivität [a] des Messions wird durch die Nernst Gleichung beschrieben:

$$EMF = E_o + 2.303 \; R \; T \; / \; (z \; F) \; \log \; [a]$$

R ist die Gaskonstante, T die absolute Temperatur, F die Faradaykonstante und z die Ladungszahl des Messions. Aus der Nernstgleichung ist ersichtlich, dass das Messsignal temperaturabhängig ist. Innerhalb physiologischer Konzentrationsbereiche ändern sich in Abhängigkeit der Temperatur, insbesondere bei in wässrigen Flüssigkeiten gelösten Alkalimetallkationen, die Konzentrationswerte nicht, wohl aber das potentiometrische Signal.

Ähnliches gilt für die Konzentrationswerte mancher Metabolite wie z.B. Glukose und Laktat. Je nach Messprinzip des sensorischen Elementes ist das Messsignal mehr oder weniger temperaturabhängig.

Bei sensorischen Elementen zur Bestimmung der Gase, beispielsweise der Blutgase oder des pH-Werts hängt das Messsignal in der Regel von einer Reihe temperaturabhängiger Größen ab, beispielsweise sowohl von im sensorischen Element ablaufenden Prozessen, als auch vom Analytwert der Flüssigkeit.

Die Erfindung wird im Folgenden anhand von schematischen Darstellungen und Diagrammen näher erläutert. Es zeigen:

Fig. 1 eine Vorrichtung zur Thermostatisierung einer in einen Analysator einsetzbaren Messzelle zur Durchführung des erfindungsgemäßen Verfahrens in einer Schnittdarstellung normal zur Flussrichtung der Probe;

Fig. 2 eine Schnittdarstellung einer Messzelle im Bereich des Messkanals mit Leiterbahnen und Sensorelementen;

Fig. 3 normierte Signalverläufe der Temperatur $T_n$ des thermostatisierbaren Elementes des Analysators und der Leitfähigkeit $L_n$ der Flüssigkeit im Messkanal bei einem Temperatursprung in Prozent bei guter thermischer Ankopplung;

Fig. 4 normierte Signalverläufe $T_n$ und $L_n$ gemäß Fig. 3 bei schlechter thermischer Ankopplung;

Fig. 5 normierte Signalverläufe der Temperatur $T_n$ des thermostatisierbaren Elementes und des Messsignals $S_n$ eines elektrochemischen (potentiometrischen) Kalium-Sensors (bestehend aus einer Messelektrode und einer Referenzelektrode) in Kontakt mit einer kaliumhältigen internen oder externen Flüssigkeit bei einem Temperatursprung in Prozent bei guter thermischer Ankopplung; und

Fig. 6 normierte Signalverläufe $T_n$ und $S_n$ gemäß Fig. 5 bei schlechter thermischer Ankopplung.

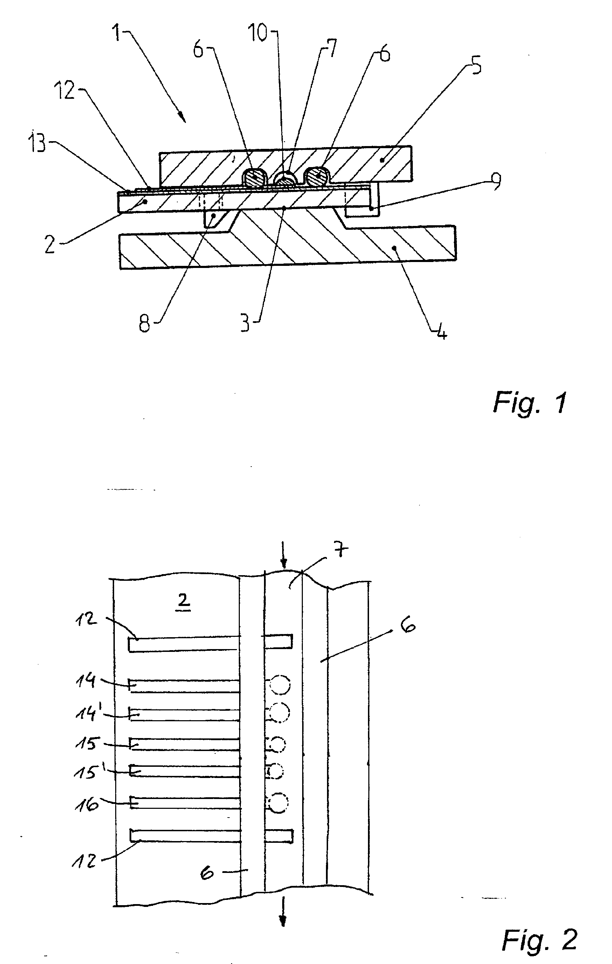

Die in Fig. 1 dargestellte Vorrichtung zur Thermostatisierung einer in einen (hier nicht weiter dargestellten) Analysator einsetzbaren Messzelle 1, weist zumindest eine planare Messzellenwand 2 auf, welche mit einer thermostatisierbaren Auflagefläche 3 des Analysators in Kontakt bringbar ist. Die Auflagefläche 3 dient zur gleichmäßigen Übertragung der von einem thermostatisierbaren Element 4 (Heiz- bzw. Kühlelement, z.B. Peltierelement) zur Verfügung gestellten Wärmeenergie.

Die Messzelle 1 ist im dargestellten Beispiel als zweiteilige Durchflusszelle ausgeführt, welche die Probe in einer Flussrichtung normal auf die Zeichnungsebene durchströmt. Die planare Messzellenwand 2 ist als Gehäuseunterteil ausgeführt, besteht aus einem gut wärmeleitenden Material und begrenzt mit einem thermisch isolierenden Gehäuseoberteil 5 - unter Zwischenlage von Dichtelementen 6- den Messkanal 7. Die beiden Gehäuseteile 2, 5 sind mit Hilfe der Rastelemente 8, 9 verbunden. Im Messkanal 7 ist zumindest ein Sensorelement 10, beispielsweise ein elektrochemischer Sensor, angeordnet. Im dargestellten Beispiel besteht die planare Messzellenwand 2 aus einem Metall oder einer Metalllegierung, sodass ein guter Wärmeübergang zu den Sensorelementen 10 und der Probe im Messkanal 7 gewährleistet ist. Bei Verwendung elektrochemischer Sensoren sind diese und deren Leiterbahnen 12 zur Ableitung der Sensorsignale unter Zwischenlage einer elektrischen Isolierung 13 an der Messzellenwand 2 angeordnet.

Zur Durchführung des erfindungsgemäßen Verfahrens wird von einem Heiz- bzw. Kühlelement die Auflagefläche 3 sprunghaft erwärmt oder gekühlt.

Die Methode der Erfassung der elektrischen Leitfähigkeit zur Bestimmung des Hämatokrit von Blutproben und die Berechnung des Hämatokrit über die gemessene Leitfähigkeit von Blutproben ist beispielsweise aus der US 4,686,479 A bekannt.

Eine derartige Anordnung zur Messung der Leitfähigkeit (siehe Fig. 2) wird erfindungsgemäß verwendet, um die temperaturbedingte Änderung der Leitfähigkeit der Flüssigkeit (z.B. Proben-, Kalibrier-, Qualitätskontroll- oder Waschflüssigkeit) im Messkanal 7 zu erfassen. Weiters dargestellt sind die auf der Messzellenwand 2, ggf. unter Zwischenlage einer elektrischen Isolierung aufgetragenen Leiterbahnen 12 zur Messung der Leitfähigkeit, sowie die Leiterbahnen 14, 14', 15, 15' und 16 für die $O_2$ -, die $CO_2$ - und die pH - Messung.

Die Qualität der thermischen Ankopplung der Messzellenwand 2 mit der Auflagefläche 3 des thermostatisierbaren Elementes 4 des Analysators ist aus der graphischen Darstellung der Temperaturkinetik ersichtlich, z.B. dadurch, dass die maximale Differenz D zwischen dem normierten Temperaturverlauf $T_n$ des thermostatisierbaren Elementes und dem normierten Verlauf der Leitfähigkeit $L_n$, des Widerstandes oder der Impedanz ermittelt wird, wobei in Abhängigkeit eines vorbestimmten Schwellwertes (beispielsweise 45%) der maximalen Differenz D die Qualität der thermischen Ankopplung beurteilt wird. Das Differenzsignal wird in den Fig. 3 bis Fig. 6 mit $D_s$ bezeichnet.

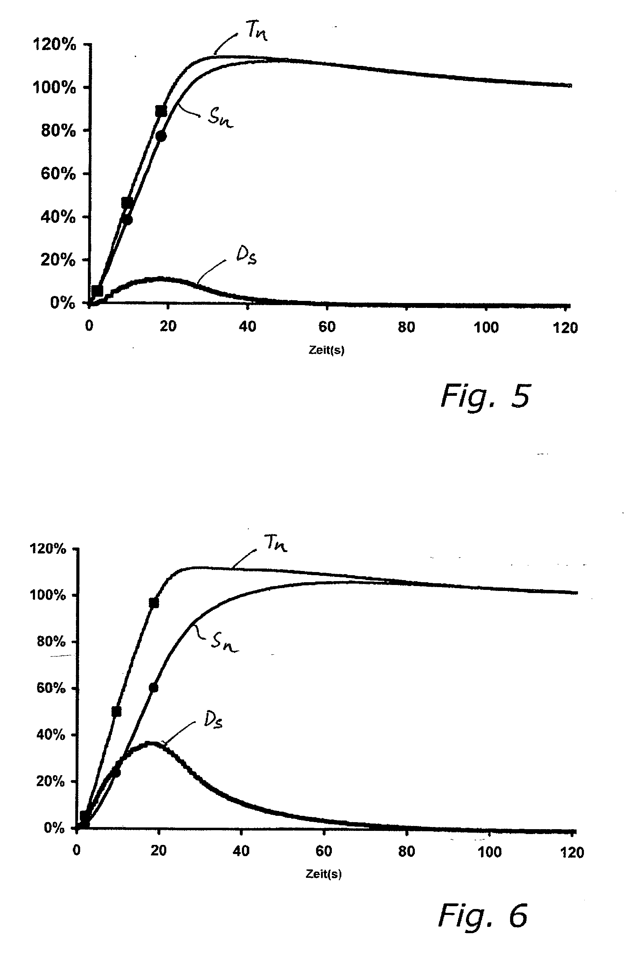

Der normierte Temperaturverlauf $T_n$ der Auflagefläche (Fig. 3 bis Fig. 6) und der normierte Signalverlauf $L_n$ der Elektroden zur Leitfähigkeitsmessung (Fig. 3 bis Fig. 4) sowie der normierte Signalverlauf $S_n$ der Kaliumelektrode (Fig. 5 bis Fig. 6) wurden wie folgt ermittelt:

In einem ersten Schritt wurden sämtliche Werte $W_t$ der Messkurve nach der Formel $W_t' = (W_t/W_{t=0})-1$ umgerechnet. W steht für den Messwert. t steht für den Zeitpunkt t. In einem zweiten Schritt wurden dann sämtliche Werte $W_t'$ nach der Formel $W_t''=(W_t'/W_{t=120}')*100$ normiert und in Form von Kurven in den Diagrammen Fig. 3 bis 6 abgebildet.

Die in den Diagrammen eingetragenen Differenzkurven $D_s$ wurden jeweils aus den Differenzwerten der beiden normierten Kurven erhalten.

Fig. 3 zeigt die Kinetik einer thermischen Kopplung in ausreichender Qualität z.B. erkennbar daran, dass das Maximum der Differenzkurve $D_s$ unter dem vorbestimmten Wert, in diesem Fall einem Wert von 45%, bleibt.

Fig. 4 zeigt die Kinetik einer durch ein Haar auf der Auflagefläche gestörten Ankopplung, erkennbar daran, dass das Maximum der Differenzkurve den vorbestimmten Wert, in diesem Fall einen Wert von 45%, übersteigt.

**Beispiel 1 (Leitfähigkeitsmessung):**

Bestimmung der maximalen Differenz D zwischen dem Temperaturverlauf T(t) der Auflagefläche und dem Verlauf der Leitfähigkeit L(t) der in der Messzelle befindlichen Flüssigkeit (siehe Fig. 3 und Fig. 4) :

$$(1) \qquad D = max\ [(T(t)-Ts)/(Te-Ts) - (L(t)-Ls)/(Le-Ls)]\ < 0,45$$

T(t)  Temperatur in Abhängigkeit der Zeit
Ts  Starttemperatur vor T-Sprung
Te  Endtemperatur nach T-Sprung
L(t)  Leitwert in Abhängigkeit der Zeit
Ls  Leitwert vor T-Sprung
Le  Leitwert nach T-Sprung

"max" bedeutet, dass für die Berechnung von D gemäß Gleichung (1) der Temperaturwert T(t) und Leitwert L(t) verwendet werden, die zu jenem Zeitpunkt t erhoben wurden, an dem das Differenzsignal D in den Fig. 3 bzw. Fig. 4 ein Maximum aufweist. Dieser Zeitpunkt kann z.B. direkt aus der Analyse des zeitlichen Verlaufs des in den Diagrammen Fig. 3 bzw. Fig. 4 abgebildeten Differenzsignals ermittelt werden.

Alternativ kann D ermittelt werden, indem eine Serie von Testwerten für D mit jeweils unterschiedlichen, über die Zeit erhobenen Wertepaaren T(t) und L(t), nach Gleichung (1) berechnet werden, wobei D dann der größte Wert aus der Serie der Testwerte ist.

Das Maximum der Differenzkurve gemäß Fig. 3 liegt bei 10.5 Sekunden. Aus den in Tabelle 1 eingetragenen Rohdaten für T und L ergibt sich nach Gleichung 1 für D ein Wert von 0.31. Dieser Wert ist kleiner als der vorbestimmte Schwellwert 0,45 (bzw. 45%). Somit ist die thermische Ankopplung der Messzelle in diesem Beispiel in Ordnung.

Tabelle 1:

| T(t=10.5s) | Ts | Te | L(t) | Ls | Le | D |
|---|---|---|---|---|---|---|
| 36.3 | 30.0 | 37.0 | 823 | 763 | 865 | 0.31 |

Das Maximum der Differenzkurve gemäß Fig. 4 liegt bei 11.0 Sekunden. Aus den in Tabelle 2 eingetragenen Rohdaten für T und L ergibt sich nach Gleichung 1 für D ein Wert von 0.61. Dieser Wert ist größer als der vorbestimmte Schwellwert 0.45 (bzw. 45%). Somit ist die thermische Ankopplung der Messzelle in diesem Beispiel nicht in Ordnung.

Tabelle 2:

| T(t=11s) | Ts | Te | L(t) | Ls | Le | D |
|---|---|---|---|---|---|---|
| 36.9 | 30.0 | 37.0 | 782 | 746 | 843 | 0.61 |

**Beispiel 2 (Leitfähigkeitsmessung):**

Die Bewertung der Qualität der Ankopplung kann auch dadurch erfolgen, dass die Krümmungsunterschiede des Anstieges der Temperaturkurve der Auflagefläche des thermostatisierbaren Elementes und des Anstieges des zeitlichen Verlaufes der Leitfähigkeit, des Widerstandes oder der Impedanz der Betriebsflüssigkeit in Form des Quotienten Q ermittelt und in Abhängigkeit eines wählbaren Schwellwertes des Quotienten Q die Qualität der thermischen Ankopplung beurteilt wird.

Beispielsweise kann der Quotient Q nach Gleichung (2) ermittelt werden

$$(2) \quad Q = \frac{(T_2 - T_1) / (T_3 - T_2)}{(L_2 - L_1) / (L_3 - L_2)}$$

wobei $T_1$, $T_2$ und $T_3$ die Temperatur der Auflagefläche des thermostatisierbaren Elements zu den vorbestimmten Zeitpunkten $t_1$, $t_2$ und $t_3$ bedeuten und $L_1$, $L_2$ und $L_3$ die Leitfähigkeit, den Widerstand oder die Impedanz der internen oder externen Flüssigkeit zu den vorbestimmten Zeitpunkten $t_1$, $t_2$ und $t_3$ bedeuten.

Aus den in Tabelle 3 eingetragenen Rohdaten für T und L, gemessen zu den vorbestimmten Zeitpunkten $t_1$=0.5s, $t_2$=4.5s und $t_3$=8.5s, ergibt sich nach Gleichung 2 für Q ein Wert von 1.26. Dieser Wert ist kleiner als der vorbestimmte Schwellwert von 1.5. Somit ist die thermische Ankopplung der Messzelle in Ordnung.

Tabelle 3

| $T_1$ | $T_2$ | $T_3$ | $L_1$ | $L_2$ | $L_3$ | Q |
|---|---|---|---|---|---|---|
| 30.3 | 32.6 | 35.3 | 764 | 783 | 811 | 1.26 |

Aus den in Tabelle 4 eingetragenen Rohdaten für T und L, gemessen zu den vorbestimmten Zeitpunkten $t_1$=0.5s, $t_2$=4.5s und $t_3$=8.5s, ergibt sich nach Gleichung 2 für Q ein Wert von 1.79. Dieser Wert ist größer als der vorbestimmte Schwellwert 1.5. Somit ist die thermische Ankopplung der Messzelle in diesem Beispiel nicht in Ordnung.

Tabelle 4:

| $T_1$ | $T_2$ | $T_3$ | $L_1$ | $L_2$ | $L_3$ | Q |
|---|---|---|---|---|---|---|
| 30.3 | 32.9 | 35.8 | 747 | 755 | 771 | 1.79 |

In einer alternativen Ausführungsvariante erfolgt die Bewertung der Qualität der thermischen Ankopplung der Messzellenwand 2 mit der Auflagefläche 3 des thermostatisierbaren Elementes 4 des Analysators über die Analyse der Temperaturkinetik z.B. dadurch, dass die maximale Differenz D zwischen dem normierten Temperaturverlauf des thermostatisierbaren Elementes und dem normierten Wert des Messsignals einer ionenselektiven Elektrode, z.B. einer kaliumselektiven Elektrode ermittelt wird, wobei in Abhängigkeit eines wählbaren Schwellwertes (beispielsweise 20%) der maximalen Differenz D die Qualität der thermischen Ankopplung beurteilt wird.

**Beispiel 3 (potentiometrische Messung):**

Bestimmung der maximalen Differenz D zwischen dem Temperaturverlauf T(t) der Auflagefläche und dem Verlauf des Messsignals S(t) eines in der Messzelle vorhandenen Sensorelementes (siehe Fig. 5 und Fig. 6) :

$$(3) \quad D = max \left[ (T(t)\text{-Ts})/(Te\text{-Ts}) - (S(t)\text{-Ss})/(Se\text{-Ss}) \right] < 0,45$$

| | |
|---|---|
| $T(t)$ | Temperatur in Abhängigkeit der Zeit |
| $Ts$ | Start-Temperatur vor T-Sprung |
| $Te$ | End-Temperatur nach T-Sprung |
| $S(t)$ | Messsignal der Kalium-Elektrode in Abhängigkeit der Zeit |
| $Ss$ | Messsignal der Kalium-Elektrode vor T-Sprung |
| $Se$ | Messsignal der Kalium-Elektrode nach T-Sprung |

"max" bedeutet, dass für die Berechnung von D gemäß Gleichung (3) der Temperaturwert $T(t)$ und Signalwert $S(t)$ der Elektrode verwendet werden, die zu jenem Zeitpunkt t erhoben wurden, an dem das Differenzsignal in den Diagrammen Fig. 5 bzw. Fig. 6 ein Maximum aufweist. Dieser Zeitpunkt kann z.B. direkt aus der Analyse des zeitlichen Signalverlaufs des in den Diagrammen Fig. 5 bzw. Fig. 6 abgebildeten Differenzsignals ermittelt werden.

Alternativ kann D ermittelt werden, indem eine Serie von Testwerten für D mit jeweils unterschiedlichen, über die Zeit erhobenen Wertepaaren $T(t)$ und $S(t)$, nach Gleichung (3) berechnet werden, wobei D dann der größte Wert aus der Serie der Testwerte ist.

Das Maximum der Differenzkurve gemäß Fig. 5 liegt bei 14.5 Sekunden. Aus den in Tabelle 5 eingetragenen Rohdaten für T und S ergibt sich nach Gleichung 3 für D ein Wert von 0.12. Dieser Wert ist kleiner als der vorbestimmte Schwellwert von z.B. 0.25 (bzw. 25%). Somit ist die thermische Ankopplung der Messzelle in Ordnung.

Tabelle 5:

| $T(t=14.5s)$ | Ts | Te | $S(t)$ | Ss | Se | D |
|---|---|---|---|---|---|---|
| 36.3 | 30.1 | 37.0 | 987 | 1076 | 962 | 0.12 |

Das Maximum der Differenzkurve gemäß Fig. 6 liegt bei 17.0 Sekunden. Aus den in Tabelle 6 eingetragenen Rohdaten für T und S ergibt sich nach Gleichung 3 für D ein Wert von 0.36. Dieser Wert ist größer als der vorbestimmte Schwellwert von 0.25 (bzw. 25%). Somit ist die thermische Ankopplung der Messzelle in diesem Beispiel nicht in Ordnung.

Tabelle 6:

| $T(t=17s)$ | Ts | Te | $S(t)$ | Ss | Se | D |
|---|---|---|---|---|---|---|
| 36.9 | 30.1 | 37.0 | 1007 | 1076 | 965 | 0.36 |

**Beispiel 4 (potentiometrische Messung):**

Die Bewertung der Qualität der Ankopplung kann auch dadurch erfolgen, dass die Krümmungsunterschiede des Anstieges der Temperaturkurve der Auflagefläche des thermostatisierbaren Elementes und des Anstieges des zeitlichen Verlaufes der Messsignals des potentiometrischen Sensors in Form des Quotienten Q ermittelt und in Abhängigkeit eines wählbaren Schwellwertes des Quotienten Q die Qualität der thermischen Ankopplung beurteilt wird.

Beispielsweise kann der Quotient Q nach Gleichung (4) ermittelt werden

$$(4) \quad Q = \frac{(T_2\text{-}T_1) / (T_3\text{-}T_2)}{(S_2\text{-}S_1) / (S_3\text{-}S_2)}$$

wobei $T_1$, $T_2$ und $T_3$ die Temperatur der Auflagefläche des thermostatisierbaren Elements zu den vorbestimmten Zeitpunkten $t_1$, $t_2$ und $t_3$ bedeuten und wobei $S_1$, $S_2$ und $S_3$ das Messsignal eines potentiometrischen Sensors zu den vorbestimmten Zeitpunkten $t_1$, $t_2$ und $t_3$ bedeuten.

Aus den in Tabelle 7 eingetragenen Rohdaten für T und S, gemessen zu den vorbestimmten Zeitpunkten $t_1$=0.5s, $t_2$=4.5s und $t_3$=8.5s, ergibt sich nach Gleichung 4 für Q ein Wert von 1.14. Dieser Wert ist kleiner als der vorbestimmte Schwellwert von 1.20. Somit ist die thermische Ankopplung in Ordnung.

Tabelle 7:

| $T_1$ | $T_2$ | $T_3$ | $S_1$ | $S_2$ | $S_3$ | Q |
|---|---|---|---|---|---|---|
| 30.1 | 31.4 | 32.9 | 1074 | 1058 | 1037 | 1.14 |

Aus den in Tabelle 8 eingetragenen Rohdaten für T und S, gemessen zu den vorbestimmten Zeitpunkten $t_1$=0.5s, $t_2$=4.5s und $t_3$=8.5s, ergibt sich nach Gleichung 4 für Q ein Wert von 1.34. Dieser Wert ist kleiner als der vorbestimmte Schwellwert von 1.20. Somit ist die thermische Ankopplung nicht in Ordnung.

Tabelle 8:

| $T_1$ | $T_2$ | $T_3$ | $S_1$ | $S_2$ | $S_3$ | Q |
|---|---|---|---|---|---|---|
| 30.1 | 31.4 | 33.1 | 1076 | 1068 | 1054 | 1.34 |

## Patentansprüche

1. Verfahren zur Überprüfung der Qualität der thermischen Ankopplung einer Messzelle (1) an ein thermostatisierbares Element (4) eines Analysators, wobei die Messzelle zur Messung zumindest eines Parameters einer Probe in den Analysator austauschbar einsetzbar ist und in einem Messkanal (7) zumindest ein Sensorelement (10) aufweist, **gekennzeichnet durch** folgende Schritte:

   a) Einsetzen der Messzelle (1) in den Analysator, wobei ein mechanischer Kontakt zum thermostatisierbaren Element (4) hergestellt wird;
   b) Befüllen des Messkanals (7) mit einer vom Analysator bereitgestellten, internen Flüssigkeit, wie Kalibrierflüssigkeit, Waschlösung, etc., oder mit einer externen Flüssigkeit, wie Probenflüssigkeit, Qualitätskontrollflüssigkeit, etc.;
   c) Abwarten eines Temperaturausgleichs zwischen interner oder externer Flüssigkeit und Messkanal;
   d) Applizieren einer sprunghaften Temperaturänderung mit Hilfe des thermostatisierbaren Elementes (4),
   e) Messen eines zeitlichen Signalverlaufes an dem zumindest einen Sensorelement (10) nach dem Applizieren der sprunghaften Temperaturänderung, wobei **durch** das Sensorelement (10) nicht die tatsächliche Temperatur bestimmt wird, sondern der zeitliche Signalverlauf einer temperaturabhängigen Größe der internen oder der externen Flüssigkeit oder der zeitliche Signalverlauf eines temperaturabhängigen, elektrochemischen oder optischen Sensorelements (10) gemessen wird; und
   f) Bestimmung der Qualität der thermischen Ankopplung aus einer Analyse des zeitlichen Signalverlaufes der Messung gemäß Punkt e).

2. Verfahren nach Anspruch 1, **dadurch gekennzeichnet, dass** zur Messung des zeitlichen Signalverlaufs gemäß Punkt e) die für die Probenmessung bereits vorhandenen Sensorelemente (10) der Messzelle (1) eingesetzt werden.

3. Verfahren nach Anspruch 1 oder 2, **dadurch gekennzeichnet, dass** in Punkt e) der zeitliche Signalverlauf einer temperaturabhängigen Größe der internen oder der externen Flüssigkeit gemessen wird.

4. Verfahren nach einem der Ansprüche 1 bis 3, **dadurch gekennzeichnet, dass** der zeitliche Signalverlauf der Leitfähigkeit, des Widerstandes oder der Impedanz der internen oder externen Flüssigkeit gemessen wird.

5. Verfahren nach Anspruch 4, **dadurch gekennzeichnet, dass** die maximale Differenz D zwischen dem normierten Temperaturverlauf des thermostatisierbaren Elementes (4) und dem normierten Verlauf der Leitfähigkeit, des Widerstandes oder der Impedanz ermittelt wird, wobei in Abhängigkeit eines wählbaren Schwellwertes der maximalen Differenz D die Qualität der thermischen Ankopplung beurteilt wird.

6. Verfahren nach Anspruch 4, **dadurch gekennzeichnet, dass** der Quotient Q des Anstieges der Temperaturkurve des thermostatisierbaren Elementes (4) und des Anstieges des zeitlichen Verlaufes der Leitfähigkeit, des Widerstandes oder der Impedanz der Betriebsflüssigkeit ermittelt und in Abhängigkeit eines wählbaren Schwellwertes des Quotienten Q die Qualität der thermischen Ankopplung beurteilt wird.

7. Verfahren nach Anspruch 1 oder 2, **dadurch gekennzeichnet, dass** in Punkt e) der zeitliche Signalverlauf eines temperaturabhängigen, elektro-chemischen oder optischen Sensorelements (10) gemessen wird.

8. Verfahren nach Anspruch 7, **dadurch gekennzeichnet, dass** der zeitliche Signalverlauf zumindest eines elektro-chemischen oder optischen Sensorelements (10) zur Bestimmung von Ionen, beispielsweise Kationen wie $Li^+$, $Na^+$, $K^+$, $Mg^{++}$, $Ca^{++}$ oder Anionen wie $Cl^-$, gemessen wird.

9. Verfahren nach Anspruch 7, **dadurch gekennzeichnet, dass** der zeitliche Signalverlauf zumindest eines elektro-chemischen oder optischen Sensorelements (10) zur Bestimmung biochemischer Substanzen wie z.B. Glukose, Laktat, Harnstoff, Creatinin, gemessen wird.

10. Verfahren nach Anspruch 7, **dadurch gekennzeichnet, dass** der zeitliche Signalverlauf zumindest eines elektro-chemischen oder optischen Sensorelements (10) zur Bestimmung von gelösten Gasen wie z.B. Sauerstoff und Kohlendioxid oder zur Bestimmung des pH-Werts, gemessen wird.

11. Verfahren nach einem der Ansprüche 7 bis 10, **dadurch gekennzeichnet, dass** die maximale Differenz D zwischen dem normierten Temperaturverlauf des thermostatisierbaren Elementes (4) und dem normierten Signalverlauf des elektrochemischen oder optischen Sensorelements (10) ermittelt wird, wobei in Abhängigkeit eines wählbaren Schwellwertes der maximalen Differenz D die Qualität der thermischen Ankopplung beurteilt wird.

12. Verfahren nach einem der Ansprüche 7 bis 10, **dadurch gekennzeichnet, dass** der Quotient Q des Anstieges der Temperaturkurve des thermostatisierbaren Elementes (4) und des Anstieges des zeitlichen Verlaufes des Messsignals des elektrochemischen oder optischen Sensorelements (10) ermittelt und in Abhängigkeit eines wählbaren Schwellwertes des Quotienten Q die Qualität der thermischen Ankopplung beurteilt wird.

**Claims**

1. Method for checking the quality of thermal coupling between a measuring cell (1) and a thermostatted element (4) of an analyser, the measuring cell being exchangeably insertable into the analyser to measure at least one parameter of a sample and being provided with at least one sensor element (10) in a measuring channel (7), **characterised by** the following steps:

   (a) inserting the measuring cell (1) into the analyser, thereby establishing mechanical contact with the thermostatted element (4);
   (b) filling the measuring channel (7) with an internal fluid supplied by the analyser, such as a calibrating fluid, rinsing fluid, etc., or with an externally supplied fluid, such as a sample fluid, quality control fluid, etc.;
   (c) waiting until temperature equilibrium is attained between the measuring channel and the internal or external fluid;
   (d) applying a rapid temperature change by means of the thermostatted element (4);
   (e) measuring the signal curve over time of the at least one sensor element (10) after the rapid temperature change has been applied, where the sensor element (10) will not determine the actual temperature, but the signal curve over time of a temperature dependent property of the internal or external fluid or the signal curve over time of a temperature dependent electrochemical or optical sensor element (10) will be measured; and
   (f) determining the quality of thermal coupling by analysing the signal curve over time resulting from the measurements of step (e).

2. Method according to claim 1, **characterised in that** sensor elements (10) already present in the measuring cell (1) for sample measurement are used for measuring the signal curve over time under item (e) above.

3. Method according to claim 1 or 2, **characterised in that** under item (e) there is measured the signal curve over time of a temperature dependent property of the internal or external fluid.

4. Method according to any of claims 1 to 3, **characterised in that** the signal curve over time of conductivity, resistance or impedance of the internal or external fluid is measured.

5. Method according to claim 4, **characterised in that** the maximum difference D between the normalised temperature curve of the thermostatted element (4) and the normalised conductivity, resistance or impedance curve is determined, and the quality of thermal coupling is evaluated using a preselected threshold value for the maximum difference D.

6.  Method according to claim 4, **characterised in that** the quotient Q of the slope of the temperature curve of the thermostatted element (4) and of the slope of the conductivity, resistance, or impedance curve of the operational fluid is determined and the quality of thermal coupling is evaluated using a preselected threshold value for the quotient Q.

7.  Method according to claim 1 or 2, **characterised in that** the signal curve over time of a temperature dependent electrochemical or optical sensor element (10) is measured under item (e) of claim 1.

8.  Method according to claim 7, **characterised in that** there is measured the signal curve over time of at least one electrochemical or optical sensor element (10) for the determination of ions, for instance cations such as $Li^+$, $Na^+$, $K^+$, $Mg^{++}$, $Ca^{++}$ or anions such as $Cl^-$.

9.  Method according to claim 7, **characterised in that** there is measured the signal curve over time of at least one electrochemical or optical sensor element (10) for the determination of biochemical substances, such as glucose, lactate, urea, creatinine.

10. Method according to claim 7, **characterised in that** there is measured the signal curve over time of at least one electrochemical or optical sensor element (10) for the determination of dissolved gases, such as oxygen or carbon dioxide, or for determining the pH-value.

11. Method according to any of claims 7 to 10, **characterised in that** the maximum difference D between the normalised temperature curve of the thermostatted element (4) and the normalised signal curve of the electrochemical or optical sensor element (10) is computed, and the quality of thermal coupling is evaluated using a preselected threshold value for the maximum difference D.

12. Method according to any of claims 7 to 10, **characterised in that** the quotient Q of the slope of the temperature curve of the thermostatted element (4) and the slope of the measurement signal curve over time of the electrochemical or optical sensor element (10) is computed, and the quality of thermal coupling is evaluated using a preselected threshold value for the quotient Q.

**Revendications**

1.  Procédé de surveillance de la qualité du couplage thermique d'une cellule de mesure (1) au niveau d'un élément réglable par thermostat (4) d'un analyseur, la cellule de mesure pouvant être utilisée de manière interchangeable dans l'analyseur pour mesurer au moins un paramètre d'un échantillon et étant pourvue d'au moins un élément capteur (10) dans un canal de mesure (7), **caractérisé par** les étapes suivantes :

    a) introduction de la cellule de mesure (1) dans l'analyseur, un contact mécanique avec l'élément réglable par thermostat (4) étant établi ;
    b) remplissage du canal de mesure (7) avec un liquide interne fourni par l'analyseur, tel un fluide d'étalonnage, une solution de lavage, etc., ou avec un liquide externe, tel un échantillon de liquide, un liquide de contrôle de qualité, etc. ;
    c) attente d'un équilibrage de la température entre le liquide interne ou externe et le canal de mesure ;
    d) application d'une variation de température discontinue à l'aide de l'élément réglable par thermostat (4) ;
    e) mesure d'une courbe de signal dans le temps au niveau de l'au moins un élément capteur (10) après l'application de la variation de température discontinue, l'élément capteur (10) ne déterminant pas la température effective, mais la courbe de signal dans le temps d'une grandeur du liquide interne ou externe dépendante de la température ou la courbe de signal dans le temps d'un élément capteur électrochimique ou optique (10) dépendant de la température ; et
    f) détermination de la qualité du couplage thermique à partir d'une analyse de la courbe de signal dans le temps de la mesure selon le point e).

2.  Procédé selon la revendication 1, **caractérisé en ce que**, pour mesurer la courbe de signal dans le temps selon le point e), on utilise les éléments capteurs (10) de la cellule de mesure (1) déjà présents pour la mesure de l'échantillon.

**3.** Procédé selon la revendication 1 ou 2, **caractérisé en ce que**, dans le point e), on mesure la courbe de signal dans le temps d'une grandeur du liquide interne ou externe dépendante de la température.

**4.** Procédé selon l'une des revendications 1 à 3, **caractérisé en ce que** la courbe de signal dans le temps de la conductivité, de la résistance ou de l'impédance du liquide interne ou externe est mesurée.

**5.** Procédé selon la revendication 4, **caractérisé en ce que** la différence maximale D entre la courbe de température normalisée de l'élément réglable par thermostat (4) et la courbe normalisée de la conductivité, de la résistance ou de l'impédance est déterminée, la qualité du couplage thermique étant appréciée en fonction d'une valeur seuil sélectionnable de la différence maximale D.

**6.** Procédé selon la revendication 4, **caractérisé en ce que** le quotient Q de la pente de la courbe de température de l'élément réglable par thermostat (4) et de la pente de la courbe dans le temps de la conductivité, de la résistance ou de l'impédance du liquide de service est déterminé et la qualité du couplage thermique est appréciée en fonction d'une valeur seuil sélectionnable du quotient Q.

**7.** Procédé selon la revendication 1 ou 2, **caractérisé en ce que**, dans le point e), la courbe de signal dans le temps d'un élément capteur électrochimique ou optique (10) dépendant de la température est mesurée.

**8.** Procédé selon la revendication 7, **caractérisé en ce que** la courbe de signal dans le temps d'au moins un élément capteur électrochimique ou optique (10) est mesurée pour le dosage d'ions, par exemple des cations tels que $Li^+$, $Na^+$, $K^+$, $Mg^{++}$, $Ca^{++}$, ou des anions tels que $Cl^-$.

**9.** Procédé selon la revendication 7, **caractérisé en ce que** la courbe de signal dans le temps d'au moins un élément capteur électrochimique ou optique (10) est mesurée pour le dosage de substances biochimiques telles que le glucose, le lactate, l'urée, la créatinine, par exemple.

**10.** Procédé selon la revendication 7, **caractérisé en ce que** la courbe de signal dans le temps d'au moins un élément capteur électrochimique ou optique (10) est mesurée pour le dosage de gaz dissous tels que l'oxygène et le dioxyde de carbone, par exemple, ou pour la détermination du pH.

**11.** Procédé selon l'une des revendications 7 à 10, **caractérisé en ce que** la différence maximale D entre la courbe de température normalisée de l'élément réglable par thermostat (4) et la courbe de signal normalisée de l'élément capteur électrochimique ou optique (10) est déterminée, la qualité du couplage thermique étant appréciée en fonction d'une valeur seuil sélectionnable de la différence maximale D.

**12.** Procédé selon l'une des revendications 7 à 10, **caractérisé en ce que** le quotient Q de la pente de la courbe de température de l'élément réglable par thermostat (4) et de la pente de la courbe dans le temps du signal de mesure de l'élément capteur électrochimique ou optique (10) est déterminé et la qualité du couplage thermique est appréciée en fonction d'une valeur seuil sélectionnable du quotient Q.

*Fig. 1*

*Fig. 2*

Fig. 3

Fig. 4

Fig. 5

Fig. 6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5046496 A
- US 6890757 B2
- US 20030057108 A1
- EP 1367392 B1
- EP 1674866 A
- US 4686479 A